# EUROPEAN PATENT APPLICATION

(11) **EP 2 505 139 A1**
(43) Date of publication of application: **03.10.2012**
(21) Application number: 12161501.7
(22) Date of filing: 27.03.2012
(51) Int. Cl.: A61B 5/145

(54) **implantable sensor**

(30) Priority: 29.03.2011 JP 2011072310
(71) Applicant: Terumo Kabushiki Kaisha, Tokyo 151-0072 (JP); Olympus Corporation, Shibuya-ku Tokyo 151-0072 (JP)
(72) Inventor: Chin, Takashi, Ashigarakami-gun, Kanagawa 259-0151 (JP); Matsumoto, Atsushi, Ashigarakami-gun, Kanagawa 259-0151 (JP); Tokimoto, Takahira, Shibuya-ku, Tokyo 151-0072 (JP)
(74) Representative: TBK

(57) **Abstract**

An embeddable sensor (10) includes a sensor body (12), a detector (14) housed in the sensor body (12), and a retaining mechanism (16) mounted on a side surface of the sensor body (12). The retaining mechanism (16) includes at least one projection (22a, 22b) on the side surface of the sensor body (12). The projection (22a, 22b) is of a V shape which is progressively wider and higher from a distal end toward a proximal end of the sensor body (12). The projection (22a, 22b) is made of a material whose hardness is lowered upon elapse of a certain period of time within a living body (S). When the embeddable sensor (10) is inserted in the living body (S), the projection (22a, 22b) retains the embeddable sensor (10) within the living body (S). Upon elapse of the certain period of time within the living body (S), the projection (22a, 22b) loses its capability to retain the embeddable sensor (10) within the living body (S).

## Description

### BACKGROUND OF THE INVENTION

### Field of the Invention:

The present invention relates to a sensor, which can be embedded in a living body, for detecting an analyte. Description of the Related Art:
Heretofore, it has been customary in medical tests to dwell or embed a sensor in the living body of a test subject (user) for detecting an analyte in the blood or body fluid of the test subject, e.g., a blood glucose level in the blood. As disclosed in Japanese Patent No. 4065575, for example, the sensor is loaded in an insertion device that has an insertion needle for puncturing a human skin. The insertion needle with the sensor loaded therein is inserted into the test subject, and the sensor is dwelled or embedded in a desired position in the living body of the test subject. Thereafter, only the insertion needle is removed from the living body, leaving the sensor dwelling in the desired position.

### SUMMARY OF THE INVENTION

However, when the insertion needle is removed from the living body after dwelling the sensor in the living body by the insertion needle, the sensor may possibly also be removed together with the insertion needle. If the sensor is also removed from the living body, the sensor may need to be inserted again into the living body or a new sensor may have to be used for insertion into the living body. The process of re-inserting the removed sensor into the living body or inserting a new sensor into the living body is tedious and time-consuming, and tends to place a burden on the test subject.

It is a general object of the present invention to provide a sensor which is prevented from being removed from a living body when an insertion needle is removed after placing the sensor in the living body, and which is capable of reliably dwelling in the living body after being inserted into the living body.

According to the present invention, there is provided a sensor adapted to be loaded in an insertion needle and inserted with the insertion needle into a living body for detecting an analyte, comprising a sensor body, a detector disposed in the sensor body, for detecting the analyte, and a retaining mechanism mounted on the sensor body, for retaining the sensor body in the living body against movement along a direction which is opposite to a direction along which the sensor body is inserted into the living body, wherein the retaining mechanism is made of a material whose hardness is lowered with time in the living body or a biodegradable material.

As described above, the sensor which is adapted to be loaded in the insertion needle and inserted with the insertion needle into the living body for detecting an analyte, includes the retaining mechanism on the sensor body which houses the detector therein, for retaining the sensor body in the living body against movement along the direction which is opposite to the direction along which the sensor body is inserted into the living body with the insertion needle. Specifically, after the sensor which is loaded in an insertion needle is inserted into the living body, the insertion needle is removed from the living body. At this time, the retaining mechanism securely retains the sensor body in the living body against removal together with the insertion needle from the living body.

The period of time in which the biodegradable material is decomposed in the living body can be adjusted based on the composition of the biodegradable material and the process by which it is manufactured. Therefore, the retaining mechanism can be hard enough to keep the sensor body in situ within the living body against removal when the sensor is inserted in the living body, and can be decomposed or softened for allowing the sensor body to be removed easily from the living body in the event that the sensor needs to be removed after use or that the sensor suffers malfunctioning or some trouble during use.

Since only the insertion needle is removed from the living body after placing the sensor in the living body, the sensor is not removed together with the insertion needle from the living body and it is not necessary to reinsert the sensor. As the sensor does not need to be reinserted into the living body, there is no undue burden imposed on the living body. The sensor thus kept dwelling within the living body can efficiently and reliably detect a desired analyte in the living body.

The above and other objects, features, and advantages of the present invention will become more apparent from the following description when taken in conjunction with the accompanying drawings in which preferred embodiments of the present invention are shown by way of illustrative example.

### BRIEF DESCRIPTION OF THE DRAWINGS

FIG. 1 is a perspective view of an embeddable sensor according to a first embodiment of the present invention;
FIG. 2 is a cross-sectional view taken along line II - II of FIG. 1;
FIG. 3 is a side elevational view of the embeddable sensor shown in FIG. 1;
FIG. 4 is a plan view of the embeddable sensor shown in FIG. 1;
FIG. 5 is a view, partly in cross section, of the embeddable sensor shown in FIG. 1 and an insertion device for inserting the embeddable sensor into a living body;
FIG. 6A is a view, partly in cross section, showing the manner in which the embeddable sensor shown in FIG. 5 and an insertion needle are inserted in the living body;
FIG. 6B is a view, partly in cross section, showing the manner in which the insertion needle is removed from the living body and the embeddable sensor is left dwelling in the living body;
FIG. 6C is a view, partly in cross section, showing the manner in which projections of the embeddable sensor are dissolved away;
FIG. 7A is a transverse cross-sectional view of an embeddable sensor according to a second embodiment of the present invention;
FIG. 7B is a side elevational view of the embeddable sensor shown in FIG. 7A;
FIG. 8A is a transverse cross-sectional view of an embeddable sensor according to a third embodiment of the present invention; and
FIG. 8B is a side elevational view of the embeddable sensor shown in FIG. 8A.

### DESCRIPTION OF THE PREFERRED EMBODIMENTS

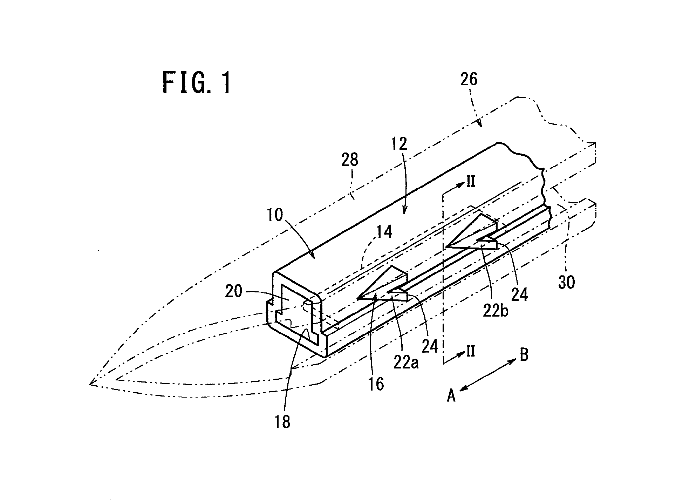

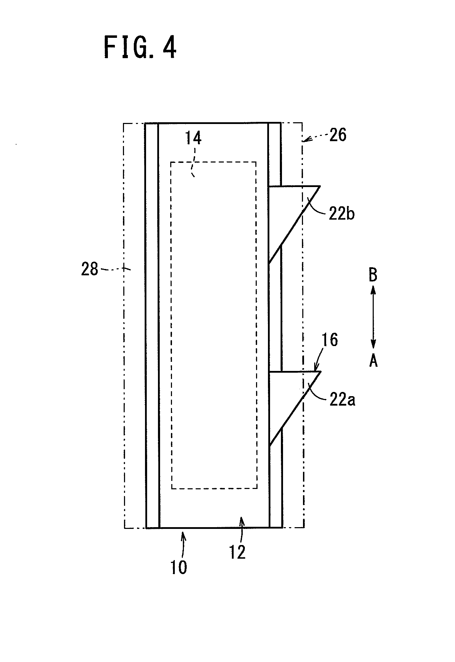

FIGS. 1 to 5 show an embeddable sensor 10 according to a first embodiment of the present invention.

As shown in FIGS. 1 to 5, the embeddable sensor 10 includes a tubular sensor body 12, a detector 14 disposed in the sensor body 12, an electric unit 15 for receiving, processing data, and sending signals, and a retaining mechanism 16 mounted on a side surface of the sensor body 12.

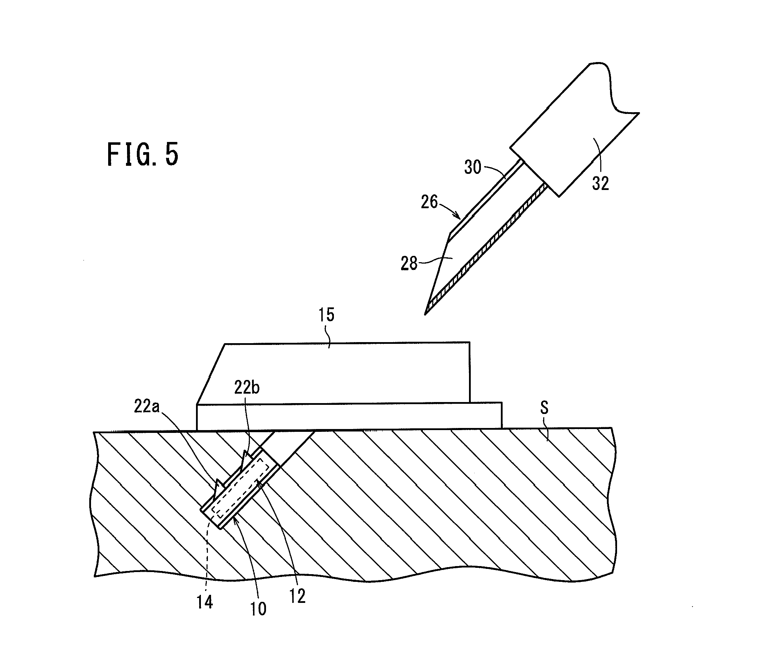

As shown in FIG. 1, the sensor body 12 has a left end, referred to as a "distal end", with respect to the direction along which the embeddable sensor 10 is inserted into a living body S (see FIG. 5), i.e., the direction indicated by the arrow A, and a right end, referred to as a "proximal end", with respect to the direction indicated by the arrow B. The distal end and the proximal end thus defined will also be used throughout all the other figures of the drawings.

The sensor body 12 has a substantially rectangular cross-sectional shape including a storage area 18 formed therein which extends axially all the way through the sensor body 12 along the directions indicated by the arrows A, B. The storage area 18 houses therein the detector 14 which detects analytes such as components including glucose, uric acid, cholesterol, protein, minerals, etc. contained in the body fluid (e.g., the blood), and also pH, microorganisms, enzymes, etc., for example. The detector 14 is securely held in the storage area 18 by a sealant 20 made of a resin material. The sealant 20 encloses electric leads, not shown, therein.

The electric unit 15 is electrically connected to the detector 14 by the electric means (leads or the like). The electric unit 15 receives signals from the detector 14, processes the received signals, and sends the processed signals to a signal receiver, not shown.

The retaining mechanism 16 comprises at least one projection, e.g., two projections 22a, 22b, mounted on a side surface of the sensor body 12 which lies along the axial direction of the sensor body 12, i.e., in the directions indicated by the arrows A, B. The projections 22a, 22b project laterally from the side surface of the sensor body 12. Each of the projections 22a, 22b is of a V shape progressively wider and higher from the distal end toward the proximal end of the sensor body 12, i.e., along the direction indicated by the arrow B.

Stated otherwise, each of the projections 22a, 22b is of a V shape progressively higher and wider toward the proximal end of the embeddable sensor 10, i.e., along the direction indicated by the arrow B which is opposite to the direction along which the embeddable sensor 10 is inserted into the living body S, i.e., the direction indicated by the arrow A. The projections 22a, 22b are spaced from each other along the axial directions of the sensor body 12, i.e., along the directions indicated by the arrows A, B.

Each of the projections 22a, 22b has an end face on its proximal end which faces in the direction indicated by the arrow B, functioning as an engaging surface 24 for retaining the sensor body 12 in the living body S (see FIG. 5) against removal therefrom. The engaging surfaces 24 of the projections 22a, 22b lie substantially perpendicularly to the axial directions of the sensor body 12, i.e., the directions indicated by the arrows A, B.

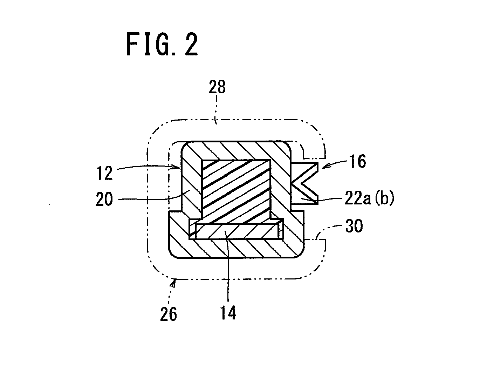

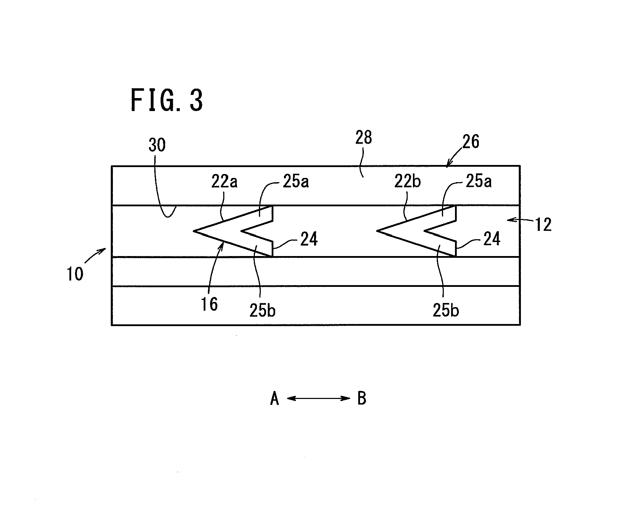

As shown in FIGS. 2 and 3, each of the projections 22a, 22b includes portions that are of a triangular cross sectional shape tapered in the axial direction (the direction indicated by the arrow A) of the sensor body 12 as viewed from the distal end or proximal end of the sensor body 12. Specifically, each of the projections 22a, 22b has two walls 25a, 25b that are progressively spaced away from each other along the direction from the distal end toward the proximal end of the sensor body 12 and are inclined to the outside of the side surface of the sensor body 12 along the direction from the proximal end toward the distal end of the sensor body 12.

The projections 22a, 22b are made of a highly biocompatible material (or biocompatible polymeric material) such as polyacrylamide or the like. When the highly biocompatible material is dry right after the embeddable sensor 10 is inserted in the living body S, it is hard enough to retain the embeddable sensor 10 within the living body S. When the highly biocompatible material absorbs water in the living body S, it becomes gradually softer. Upon elapse of a certain period of time, e.g., several minutes, after the embeddable sensor 10 has been inserted in the living body S, the projections 22a, 22b becomes soft enough to allow the embeddable sensor 10 to be removed from the living body S. If the projections 22a, 22b are made of a material that is safe for the living body S (or a biodegradable material), e.g., sucrose, caramel, or the like, then they are gradually dissolved when they absorb water within the living body S, and are completely dissolved away upon elapse of a certain period of time after the embeddable sensor 10 has been inserted in the living body S.

The projections 22a, 22b may be made of a material whose hardness varies depending on a change in the ambient temperature, including gelatin or the like. If the projections 22a, 22b are made of such a material, then when the embeddable sensor 10 is outside of the living body S, the projections 22a, 22b keep a certain level of hardness, and when the embeddable sensor 10 is inserted into the living body S, the projections 22a, 22b are heated by the temperature of the living body S, gradually reducing their hardness.

An insertion needle 28 of a sensor inserting device 26 for inserting the embeddable sensor 10 into the living body S will briefly be described below with reference to FIGS. 1 and 5.

The insertion needle 28 has a generally rectangular or circular cross-sectional shape or the like. Specifically, the insertion needle 28 includes an open groove 30 formed in and extending along a side wall thereof. As a result, the insertion needle 28 is cross-sectionally U-shaped, C-shaped, V-shaped or the like. The insertion needle 28 has a proximal end integral with a grip 32 which is gripped to insert the embeddable sensor 10 into the living body S. The embeddable sensor 10 is inserted into the insertion needle 28 from its distal end with the projections 22a, 22b moving in and along the open groove 30. When the embeddable sensor 10 is inserted in the insertion needle 28, the projections 22a, 22b project out of the open groove 30, as shown in FIG. 2. At this time, the embeddable sensor 10 stays securely in the insertion needle 28 with each other.

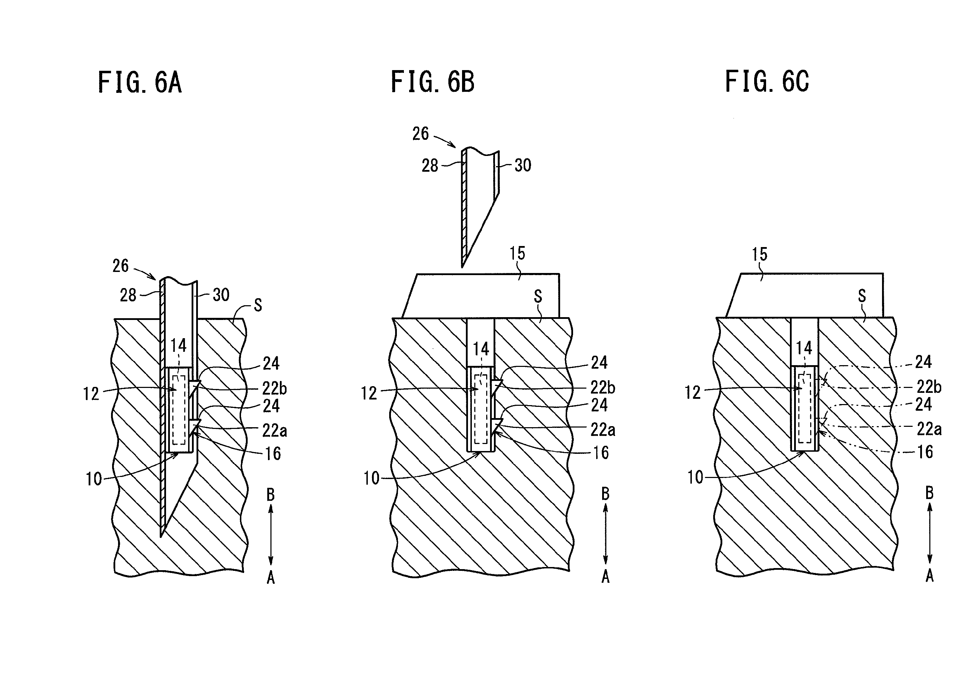

The embeddable sensor 10 according to the first embodiment of the present invention is basically constituted as described above. A process of inserting the embeddable sensor 10 into the living body S will be described below with reference to FIGS. 5 and 6A to 6C.

First, the embeddable sensor 10 is inserted into the insertion needle 28 of the sensor inserting device 26 from its distal end. Then, the insertion needle 28 is positioned so as to face a region of the test subject into which the embeddable sensor 10 is to be inserted. As shown in FIG. 6A, the insertion needle 28 is pushed to puncture the skin of the region of the test subject until the embeddable sensor 10 is placed in a desired position where it should dwell in the living body S.

After having confirmed that the embeddable sensor 10 is placed in the desired position in the living body S, the insertion needle 28 is pulled out of the living body S. At this time, the projections 22a, 22b are held in engagement with the nearby tissue of the living body S against removal along the direction indicated by the arrow B along which the insertion needle 28 is pulled out. Therefore, the embeddable needle 10 is prevented from being removed together with the insertion needle 28 out of the living body S, but remains dwelling in the desired position, as shown in FIG. 6B.

More specifically, when the embeddable sensor 10 is pulled along the direction out of the living body S, i.e., along the direction indicated by the arrow B, since the engaging surfaces 24 of the projections 22a, 22b lie substantially perpendicularly to the direction indicated by the arrow B, the projections 22a, 22b effectively engage the nearby tissue of the living body S, firmly retaining the embeddable sensor 10 against removal from the living body S.

When the insertion needle 28 is removed out of the living body S, the projections 22a, 22b of the embeddable sensor 10 are located in the open groove 30 and do not contact and hence interfere with the insertion needle 28.

While the embeddable sensor 10 is dwelling in the living body S, the detector 14 detects a desired analyte concentration, e.g., a glucose concentration, in the blood, and outputs a detection signal representative of the detected analyte concentration to the electric unit 15.

As shown in FIG. 6C, the projections 22a, 22b of the embeddable sensor 10 which is dwelling in the living body S are gradually softened with time by absorbing water or the like in the living body S, lowering their hardness. Upon elapse of a certain period of time, the engaging surfaces 24 of the projections 22a, 22b are dissolved out of engagement with the nearby tissue of the living body S. Consequently, the embeddable sensor 10 can easily be pulled out of the living body S.

Even when the projections 22a, 22b are softened, the embeddable sensor 10 does not freely move in the living body S, but is retained in situ within the tissue of the living body S.

According to the first embodiment, as described above, the embeddable sensor 10 which is embedded in the living body S to detect a blood glucose level, for example, thereof includes at least one of the projections 22a, 22b on one side surface of the sensor body 12 which houses the detector 14 therein. Each of the projections 22a, 22b is progressively higher and wider from the distal end toward the proximal end of the sensor body 12, i.e., along the direction indicated by the arrow B. Therefore, when only the insertion needle 28 is removed after the embeddable sensor 10 has been inserted together with the insertion needle 28 into the living body S, the projections 22a, 22b engage the nearby tissue of the living body S, preventing the embeddable sensor 10 from being dragged together with the insertion needle 28 out of the living body S.

The embeddable sensor 10 thus remains dwelling in the desired position in the living body S, for detecting the concentration of a desired analyte in the living body S.

The projections 22a, 22b of the embeddable sensor 10 are made of a material that is safe for the living body S, e.g., sucrose, caramel, or the like, or a highly biocompatible material such as polyacrylamide or the like. Therefore, upon elapse of a certain period of time after the embeddable sensor 10 has been placed in the living body S, the projections 22a, 22b are gradually softened with time by absorbing water in the living body S, lowering their hardness. Therefore, the projections 22a, 22b lose their capability to retain the embeddable sensor 10 within the living body S. If the embeddable sensor 10 suffers a malfunction or other trouble, therefore, the embeddable sensor 10 can easily and reliably be removed from the living body S after the hardness of the projections 22a, 22b is lowered upon elapsed of a certain period of time.

The projections 22a, 22b are progressively narrower and lower along the direction in which the embeddable sensor 10 is inserted into the living body S, i.e., along the direction indicated by the arrow A. Therefore, when the embeddable sensor 10 is inserted into the living body S, it undergoes relatively small resistance from the living body S and imposes only a small burden on the test subject.

The projections 22a, 22b have the respective engaging surfaces 24 as their end faces on the proximal ends thereof along the direction indicated by the arrow B which is opposite to the direction along which the embeddable sensor 10 is inserted into the living body S, i.e., the direction indicated by the arrow A. The engaging surfaces 24 of the projections 22a, 22b lie substantially perpendicularly to the direction in which the embeddable sensor 10 is inserted into the living body S, i.e., the axial directions of the sensor body 12, i.e., the directions indicated by the arrows A, B. Therefore, when the embeddable sensor 10 is pulled along the direction indicated by the arrow B, the engaging surfaces 24 engage the nearby tissue of the living body S, keeping the embeddable sensor 10 securely dwelling in the living body S.

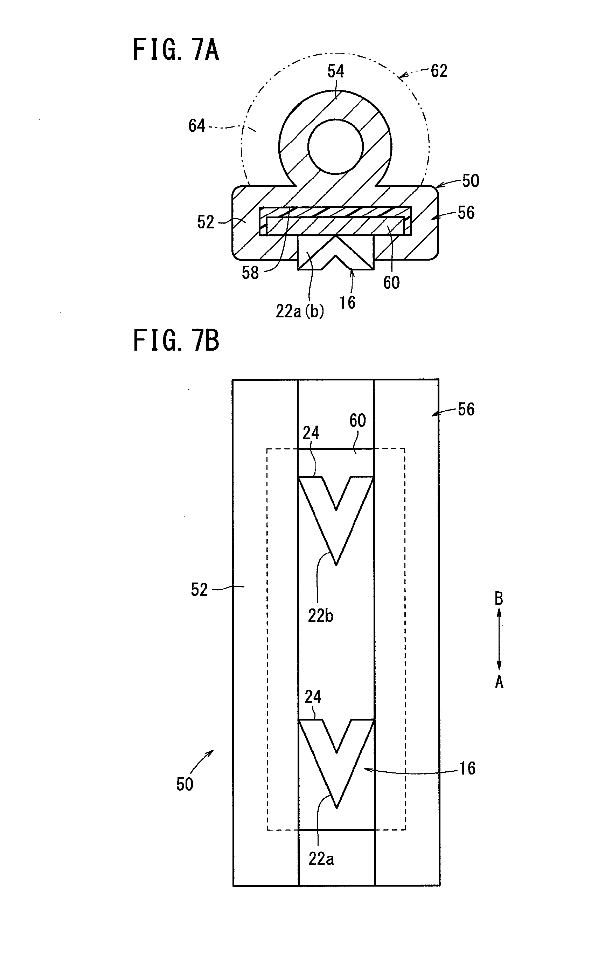

FIGS. 7A and 7B show an embeddable sensor 50 according to a second embodiment of the present invention. Those parts of the embeddable sensor 50 according to the second embodiment which are identical to those of the embeddable sensor 10 according to the first embodiment are denoted by identical reference characters, and will not be described in detail below.

As shown in FIGS. 7A and 7B, the embeddable sensor 50 according to the second embodiment includes a sensor body 56 having a base 52 and a ring 54. The base 52 has a storage area 58 formed therein which houses a detector 60 therein. The sensor body 56 also includes at least one projection, e.g., two projections 22a, 22b, mounted on and projecting from a lower surface of the base 52. As with the embeddable sensor 10 according to the first embodiment, each of the projections 22a, 22b is of a V shape progressively wider and higher from the distal end toward the proximal end of the sensor body 56, i.e., along the direction indicated by the arrow B.

A sensor inserting device 62 for inserting the embeddable sensor 50 into the living body S includes an insertion needle 64. The insertion needle 64 has a generally semicircular cross-sectional shape. The ring 54 of the sensor body 56 can be inserted and held in the insertion needle 64. When the embeddable sensor 50 is inserted and held in the distal end portion of the insertion needle 64, the base 52 of the sensor body 56 protrudes out of the insertion needle 64.

After the embeddable sensor 50 is placed together with the insertion needle 64 in the desired position in the living body S, the insertion needle 64 is pulled out of the living body S along the direction indicated by the arrow B. At this time, at least one of the projections 22a, 22b is held in engagement with the nearby tissue of the living body S against removal along the direction indicated by the arrow B. As a consequence, the embeddable sensor 50 is prevented from being removed together with the insertion needle 64 out of the living body S, but remains dwelling in the desired position. The projections 22a, 22b of the embeddable sensor 50 which is dwelling in the living body S are gradually softened with time by absorbing water or the like in the living body S, lowering their hardness. Therefore, the projections 22a, 22b lose their capability to retain the embeddable sensor 50 within the living body S. The embeddable sensor 50 can thus easily be removed from the living body S.

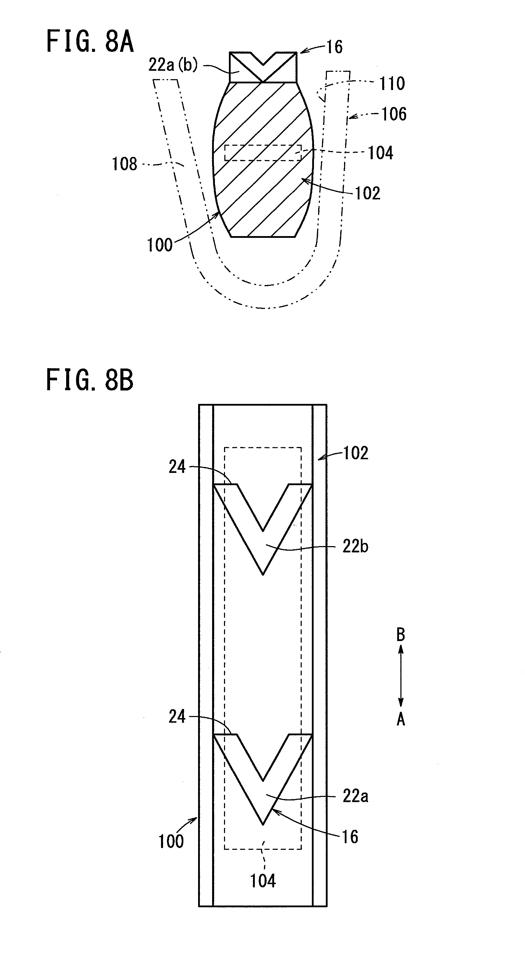

FIGS. 8A and 8B show an embeddable sensor 100 according to a third embodiment of the present invention. Those parts of the embeddable sensor 100 according to the third embodiment which are identical to those of the embeddable sensors 10, 50 according to the first and second embodiments are denoted by identical reference characters, and will not be described in detail below.

As shown in FIGS. 8A and 8B, the embeddable sensor 100 according to the third embodiment includes a sensor body 102 which is of a substantially rectangular cross-sectional shape. The sensor body 102 houses a detector 104 therein. The sensor body 102 includes at least one projection, e.g., two projections 22a, 22b, mounted on and projecting from an upper surface thereof.

As with the embeddable sensors 10, 50 according to the first and second embodiments, each of the projections 22a, 22b is of a V shape progressively wider and higher from the distal end toward the proximal end of the sensor body 102, i.e., along the direction indicated by the arrow B.

A sensor inserting device 106 for inserting the embeddable sensor 100 into the living body S includes an insertion needle 108. The insertion needle 108 has a generally U-shape cross section. The insertion needle 108 has an opening 110 formed in and extending along a side wall thereof. The sensor body 102 can be inserted and held in the insertion needle 108 with the projections 22a, 22b disposed in the opening 110 and projecting out of the opening 110.

After the embeddable sensor 100 are placed together with the insertion needle 108 in the desired position in the living body S, the insertion needle 108 is pulled out of the living body S along the direction indicated by the arrow B. At this time, at least one of the projections 22a, 22b is held in engagement with the nearby tissue of the living body S against removal along the direction indicated by the arrow B. As a consequence, the embeddable sensor 100 is prevented from being removed together with the insertion needle 108 out of the living body S, but remains dwelling in the desired position.

When the insertion needle 108 is removed out of the living body S, the projections 22a, 22b of the embeddable sensor 10 are located in the opening 110 and do not contact and hence interfere with the insertion needle 108. The projections 22a, 22b of the embeddable sensor 100 which is dwelling in the living body S are gradually softened with time by absorbing water in the living body S, lowering their hardness. Therefore, the projections 22a, 22b lose their capability to retain the embeddable sensor 100 within the living body S. The embeddable sensor 100 can thus easily be removed from the living body S.

In the embeddable sensors 10, 50, 100 according to the first, second, and third embodiments, the projections 22a, 22b are illustrated as being of a V shape which is progressively higher and wider toward the proximal end of the embeddable sensors 10, 50, 100, i.e., along the direction indicated by the arrow B which is opposite to the direction along which the embeddable sensors 10, 50, 100 are inserted into the living body S, i.e., the direction indicated by the arrow A. However, the projections 22a, 22b are not limited to any particular shapes insofar as they are progressively higher or wider along the direction opposite to the direction in which the embeddable sensors 10, 50, 100 are inserted into the living body S.

The embeddable sensors 10, 50, 100 according to the first, second, and third embodiments are illustrated as having two projections 22a, 22b. However, the embeddable sensor according to the present invention may have only one projection or three or more projections.

The principles of the present invention are not limited to an embeddable sensor that will be fully inserted in the living body S, but may be applicable to an embeddable sensor which has at least a detector 14, 60, 104 insertable in the living body S with other parts exposed outside of the living body.

An embeddable sensor (10) includes a sensor body (12), a detector (14) housed in the sensor body (12), and a retaining mechanism (16) mounted on a side surface of the sensor body (12). The retaining mechanism (16) includes at least one projection (22a, 22b) on the side surface of the sensor body (12). The projection (22a, 22b) is of a V shape which is progressively wider and higher from a distal end toward a proximal end of the sensor body (12). The projection (22a, 22b) is made of a material whose hardness is lowered upon elapse of a certain period of time within a living body (S). When the embeddable sensor (10) is inserted in the living body (S), the projection (22a, 22b) retains the embeddable sensor (10) within the living body (S). Upon elapse of the certain period of time within the living body (S), the projection (22a, 22b) loses its capability to retain the embeddable sensor (10) within the living body (S).

## Claims

1. A sensor (10, 50, 100) adapted to be loaded in an insertion needle (28) and inserted with the insertion needle (28) into a living body for detecting an analyte in the living body, comprising:
a sensor body (12, 52, 102);
a detector (14, 60, 104) disposed in the sensor body (12, 52, 102), for detecting the analyte; and
a retaining mechanism mounted on the sensor body (12, 52, 102), for retaining the sensor body (12, 52, 102) in the living body against movement along a direction which is opposite to a direction along which the sensor body (12, 52, 102) is inserted into the living body;
wherein the retaining mechanism is made of a material whose hardness is lowered with time in the living body or a biodegradable material.

2. The sensor according to claim 1, wherein the retaining mechanism comprises a projection (22a, 22b) projecting from a side surface of the sensor body (12, 52, 102), the projection being progressively higher along the direction which is opposite to the direction along which the sensor body (12, 52, 102) is inserted into the living body.

3. The sensor according to claim 1, wherein the retaining mechanism comprises a projection (22a, 22b) projecting from a side surface of the sensor body (12, 52, 102), the projection being progressively wider along the direction which is opposite to the direction along which the sensor body (12, 52, 102) is inserted into the living body.

4. The sensor according to claim 2, wherein the projection is progressively wider along the direction which is opposite to the direction along which the sensor body (12, 52, 102) is inserted into the living body.

5. The sensor according to claim 1, wherein the retaining mechanism is made of sucrose or gelatin which is safe for the living body, or a water-dissolvable biocompatible polymeric material.

6. The sensor according to claim 2, wherein the retaining mechanism is made of sucrose or gelatin which is safe for the living body, or a water-dissolvable biocompatible polymeric material.

7. The sensor according to claim 3, wherein the retaining mechanism is made of sucrose or gelatin which is safe for the living body, or a water-dissolvable biocompatible polymeric material.
